# EUROPEAN PATENT SPECIFICATION

(11) **EP 1 265 625 B1**
(45) Date of publication and mention of the grant of the patent: **22.12.2004**
(21) Application number: 01901067.7
(22) Date of filing: 10.01.2001
(51) Int. Cl.: A61K 35/78

(54) **MEDICINAL OINTMENT AND PROCESS FOR ITS PREPARATION**
MEDIZINISCHE SALBE SOWIE DEREN HERSTELLUNGSVERFAHREN
POMMADE MEDICALE ET PROCEDE DE SA PREPARATION

(30) Priority: 19.01.2000 BA 000529
(43) Date of publication of application: 18.12.2002
(73) Proprietor: Kraljevic-Bulic, Angelina, 88320 Ljubuski, Vasarovici (BA)
(72) Inventor: Kraljevic-Bulic, Angelina, 88320 Ljubuski, Vasarovici (BA)
(74) Representative: Lieck, Hans-Peter, Dipl.-Ing.
(86) International application number: PCT/BA2001/000001
(87) International publication number: WO 2001/052872

(56) References cited:
- WO-A-97/42963
- US-A- 4 699 929

## Description

The invention relates to a medicinal ointment and to a process for its preparation. In particular, the invention referres to the production of the subsidiary medicinal medicines for the treatment of heavy burns, fungous dermatosis, skin lesions originating out of different causes (decubitises, eczemas), boils, acnes, skin lesions caused by sun radiation, hemorrhoids, and also for forcing epithalization.

Some different types of pharmaceutical and subsidiary pharmaceutical preparations for the treatment of damped skin surfaces caused by burns, or microorganisms influence and lesions caused by different causes are already known. A lot of already known preparations are based on the synthetic chemicals as active substances and other different types of unguents. As a heating ointment for curing burns, there has already been known an ointment (the Roumanian invention RO 92445) which contains bismuthsubnitrate, ichtiol, zinkoxide, camphor, oleum jekoris, vitamin A and F, hydrocortison acetate, neomicin sulfate, lanoline and Vaseline; According to the PCT registration, the number of the international announcement WO98/50055, the name "OINTMENT FOR THE TREATMENT OF BURNS AND OF OTHER SKIN DISESEAS" by the inventor STOICA Felician Titus, the component parts are:

| | | |
|---|---|---|
| 1) | sunflower oil | 45...67 % (mass percentage) |
| 2) | olive oil | 15...37% |
| 3) | calendulae off, extract | 1...2 % |
| 4) | incense | 0.8...1.8 % |
| 5) | rosin | 2.5...8 % |
| 6) | white beeswax | 6.5...11.5 % |
| 7) | bismuthsubgalat | 0.7...1.8 % |
| 8) | camphor | 0.7...1.8 % |

According to the PCT registration, the number of the international announcement WO 98/43613, the name "OINTMENT FOR TREATING SUPPURATIVE LESIONS, BURNS AND TROPHIC ULCERS AT ALL STAGES, PREPARATION AND APPLICATION OF SAME" by the inventor MCHEDLIDZE Tamas Shalvovich, the component parts are:

| | | |
|---|---|---|
| 1) | beeswax | 10 - 300 parts (mass percentage) |
| 2) | pine tar | 10 - 300 |
| 3) | olive oil | 10 - 300 |
| 4) | pig - fat | 100 - 300 |

According to the PCT registration, the number of the international announcement WO97/42963, the name "GREEN OINTMENT" by the inventor SHIKHASHVILI Nino and DARSAVELIDZE Zurab, the component parts are:

| | | |
|---|---|---|
| 1) | Chelidonium majus | 15 - 25 g. |
| 2) | Plantago major | 15 - 25 g. |
| 3) | Matricaria Chamomilla | 15 - 25 |
| 4) | Achillea millefolium | 15 - 25 |
| 5) | Calendula officinalis | 15 - 25 |
| 6) | Hypericum perforatum | 15 - 25 |
| 7) | Eucalyptus globulus | 15 - 25 |
| 8) | Oleum olivarum | 1000 |
| 9) | Cera flava | 80 - 130 |

The essence of this invention is in a new composition, the way of extraction of liposoluble component of the used drugs and in the process of preparation. According to this invention, the following components are included in the combination:

| | | |
|---|---|---|
| 1. | Oleum olivarum | 1000 - 1500 g. |
| 2. | Aqua purificata | 2000 - 3000 g. |
| 3. | Adeps suillus | 800 - 1200 g. |
| 4. | Cera flava | 200 - 300 g. |
| 5. | Sambucus nigra | 100 - 150 g. |
| 6. | Sanguisorba minor scop. | 100 - 150 g. |
| 7. | Veronica officinalis | 100 - 150 g. |
| 8. | Polypodium vulgare | 100 - 150 g. |

The components extraction of the fresh picked and chopped drugs is done by the dissolved greasy components warmed (the used temperature is from 60°C - 140°C) in the duration period between 1 to 8 hours.
The speciality of the preparation procedure is that all the procedure is done in the aseptic conditions, so that greasy components and vax can dissolve and then the chopped drugs are added and the extraction in warm condition is done by constant mixing. Than the filtration (trickling) and filtrate cooling up to 80°C is done and Aqua purificata is added. The extracted mass is packed in some sterile containers and then closed hermetically and marked, or it can be laid onto sterile gauze of the required dimensions in a uniform layer and packed separately in aluminium foil welded by heat and marked.

In a glass, enamel or corrosion - resistant reactor, equipped by a double cover for heating, control and temperature regulation, the required weighed substance quantities are put into by a blender and a dosing device. After realization of the heating procedure and warmth extraction, the substance is transferred into the unit for filtration and after that the filtrate is transferred into the separator where, after the required cooling period, the required Aqua purificata quantity is dosed and after ointment isolation, it is transferred into the filling machine and later into packing machine or in other case it is laid onto a sterile gauze. The sterile gauze dimensions in cm may be about 5x5, 10x10, 10x20 and 20x40. All the procedure is done in the aseptic conditions. The final product is marked, packed into packages for transportation and stocked in a dry and dark place.

After ordinary medical treatment on the damaged skin surface, the sterile gauze which is laid by a thin layer of the medicinal ointment "HEALTHYDERM" by a sterile spatula, is applicated and then it is bandaged by a gentle bandage stretching. This procedure is done twice in a day and as a result the procedure of total epithalization without formation of keloids is ended even with the heaviest damages within 14 days.

At the application of the ready - made sterile gauze with the medicinal ointment "HEALTHYDERM", the procedure is analogous, but it is important to note that the separate packages with the sterile gauze laid by the medicinal ointment are opened immediately before the application.

The medicinal ointment "HEALTHYDERM" can be successfully applied in medical treatment of burns, chilblains, skin lesions caused by some aggressive chemical substances; ruddiness caused by sun radiation, decubitises, different types of dermatosis, acnes and hemorrhoids.

## Claims

1. Medicinal ointment "HEALTHYDERM",
**characterized by** the list of the following ointment compounds:
- Oleum olivarum 1000 - 1500 g.
- Aqua purificata 2000 - 3000 g.
- Adeps suillus 800 - 1200 g.
- Cera flava 200 - 300 g.
- Sambucus nigra 100 - 150 g.
- Sanguisorba minor scop. 100 - 150 g.
- Veronica officinalis 100 - 150 g.
- Polypodium vulgare 100 - 150 g.

2. Process for the preparation of a medicinal ointment according to claim 1, **characterized by** the liposoluble components extraction of the fresh chopped drugs by dissolved greasy components with the duration period of 1 to 8 hours at the temperature of 60°C to 140°C.

3. Process according to claim 2, **characterized in that** after the process of extraction, the filtering through glass filters is done.

4. Process according to claim 3, **characterized in that** the filtrate is isolated in a separator where cooling at 80°C is done and a necessary quantities of Aqua purificata is added.

5. Process according to claim 4, **characterized in that** the ointment dissolved in the separator is transferred into filling machines for filling and packing the marked sterile containers.

6. Process according to claim 5, **characterized in that** the medicinal ointment is transferred into the machine for ointment laying onto sterile cut out gauze, which is then packed into aluminium foil and hermetically closed by heat welding.

7. Process according to claim 6, **characterized in that** the sterile gauze dimensions in cm are about 5x5, 10x10, 10x20 and 20x40.

8. Process according to any preceding claims 2 to 7, **characterized in that** all the procedures are done in strictly aseptic conditions.

## Patentansprüche

1. Medizinische Salbe "HEALTHYDERM", **gekennzeichnet durch** die Liste der folgenden Salbenbestandteile:
- Oleum olivarum 1000 - 1500 g
- Aqua purificata 2000 - 3000 g
- Adeps suillus 800 - 1200 g
- Cera flava 200 - 300 g
- Sambucus nigra 100 - 150 g
- Sanguisorba minor scop. 100 - 150 g
- Veronica officinalis 100 - 150 g
- Polypodium vulgare 100 - 150 g

2. Verfahren zur Herstellung einer medizinischen Salbe nach Anspruch 1, **gekennzeichnet durch** die Extraktion fettlöslicher Bestandteile der klein geschnittenen frischen Arzneimittel **durch** gelöste fettartige Bestandteile mit der Dauer von 1 bis 8 Stunden bei der Temperatur von 60°C bis 140°C.

3. Verfahren nach Anspruch 2, **dadurch gekennzeichnet, dass** nach dem Prozess der Extraktion die Filterung durch Glasfilter erfolgt.

4. Verfahren nach Anspruch 3, **dadurch gekennzeichnet, dass** das Filtrat in einem Abscheider isoliert wird, wo auf 80°C abgekühlt wird und eine notwendige Menge Aqua purificata zugegeben wird.

5. Verfahren nach Anspruch 4, **dadurch gekennzeichnet, dass** die in dem Abscheider gelöste Salbe in Füllmaschinen gegeben wird, um die markierten sterilen Behälter zu füllen und zu verpacken.

6. Verfahren nach Anspruch 5, **dadurch gekennzeichnet, dass** die medizinische Salbe in die Maschine zum Aufbringen der Salbe auf sterile zugeschnittene Gaze gegeben wird, die dann in Aluminiumfolie verpackt und durch Heißverschweißen hermetisch verschlossen wird.

7. Verfahren nach Anspruch 6, **dadurch gekennzeichnet, dass** die Maße der sterilen Gaze in cm etwa 5x5, 10x10, 10x20 und 20x40 betragen.

8. Verfahren nach einem der vorhergehenden Ansprüche 2 bis 7, **dadurch gekennzeichnet, dass** alle Maßnahmen unter streng aseptischen Bedingungen durchgeführt werden.

## Revendications

1. Pommade médicinale "HEALTHYDERM", **caractérisée par** la liste des composés de pommade suivants :
- Huile d'olive (*Olea olivarum*) 1000 à 1500 g
- Eau purifiée (*Aqua purificata*) 2000 à 3000 g
- Axonge (*Adeps suillus*) 800 à 1200 g
- Cire jaune (*Cera flava*) 200 à 300 g
- Sureau noir (*Sambucus nigra*) 100 à 150 g
- Pimprenelle (*Sanguisorba minor scop.*) 100 à 150 g
- Véronique officinale (*Veronica officinalis*) 100 à 150 g
- Polypode commun (*Polypodium vulgare*) 100 à 150 g

2. Procédé de préparation d'une pommade médicinale selon la revendication 1, **caractérisé par** l'extraction des composants liposolubles des plantes médicinales fraîches hachées par des composants graisseux dissous pendant une durée dans la plage de 1 à 8 heures à une température dans la plage de 60°C à 140°C.

3. Procédé selon la revendication 2, **caractérisé en ce que**, après le processus d'extraction, on effectue un filtrage à travers des filtres en verre.

4. Procédé selon la revendication 3, **caractérisé en ce que** l'on isole le filtrat dans un séparateur dans lequel un refroidissement à 80°C est effectué et **en ce que** l'on ajoute une quantité nécessaire d'eau purifiée (*Aqua purificata*).

5. Procédé selon la revendication 4, **caractérisé en ce que** l'on transfère la pommade dissoute dans le séparateur dans des machines de conditionnement afin de remplir et conditionner les conteneurs stériles marqués.

6. Procédé selon la revendication 5, **caractérisé en ce que** l'on transfère la pommade médicinale dans la machine afin de déposer la pommade sur de la gaze stérile découpée, laquelle est ensuite conditionnée dans un film d'aluminium et fermée hermétiquement par thermosoudage.

7. Procédé selon la revendication 6, **caractérisé en ce que** les dimensions en cm de la gaze stérile sont d'environ 5 x 5, 10 x 10, 10 x 20 et 20 x 40.

8. Procédé selon l'une quelconque des revendications 2 à 7 précédentes, **caractérisé en ce que** toutes les opérations se font en conditions strictement aseptiques.
